# EUROPEAN PATENT SPECIFICATION

(11) **EP 1 221 046 B1**
(45) Date of publication and mention of the grant of the patent: **08.02.2006**
(21) Application number: 00962271.3
(22) Date of filing: 02.10.2000
(51) Int. Cl.: G01N 33/483

(54) **ASSEMBLY AND METHOD FOR DETERMINING AND/OR MONITORING ELECTROPHYSIOLOGICAL PROPERTIES OF ION CHANNELS**
ANORDNUNG UND VERFAHREN ZUM FESTSTELLEN UND/ODER ÜBERWACHEN ELEKTROPHYSIOLOGISCHER EIGENSCHAFTEN VON IONENKANÄLEN
ENSEMBLE ET PROCEDE DESTINES A DETERMINER ET/OU SURVEILLER DES PROPRIETES ELECTROPHYSIOLOGIQUES DE CANAUX IONIQUES

(30) Priority: 01.10.1999 DK 140799
(43) Date of publication of application: 10.07.2002
(73) Proprietor: Sophion Bioscience A/S, 2750 Ballerup (DK)
(72) Inventor: PETERSEN, Jon Wulff, Mikroelektronik Centret, 2800 Lyngby (DK); TELLEMAN, Pieter, Mikroelektronik Centret, 2800 Lyngby (DK); HANSEN, Ole, Mikroelektronik Centret, 2800 Lyngby (DK); CHRISTOPHERSEN, Palle, 2750 Ballerup (DK); BECH, Morten, 2750 Ballerup (DK); OLESEN, Soren, Peter, 2750 Ballerup (DK); DUE, Jorgen, 2750 Ballerup (DK); THOMSEN, Lars, 2750 Ballerup (DK)
(74) Representative: Thomas, Philip John Duval
(86) International application number: PCT/DK2000/000548
(87) International publication number: WO 2001/025769

(56) References cited:
- EP-A- 0 299 778
- EP-A- 0 299 779
- US-A- 4 062 750
- US-A- 4 225 410
- M. MULLENBORN ET AL: "Fast 3d Laser Micromachining of Silicon for Micromechanical and Microfluid Applications" , SOLID-STATE SENSORS AND ACTUATORS, 1995 AND EUROSENSORS IX.. TRANSDUCERS '95. THE 8TH INTERNATIONAL CONFERENCE ON, , STOCKHOLM, SE XP002901487 page 166 -page 169; figure 7
- WEAVER J C: "Electroporation of cells and tissues " IEEE TRANSACTIONS ON PLASMA SCIENCE, vol. 28, no. 1, February 2000 (2000-02), pages 24-33, XP002901488

## Description

### TECHNICAL FIELD

The present invention relates to a substrate and a method for determining and/or monitoring electrophysiological properties of ion channels of ion channel-containing structures, typically lipid membrane-containing structures such as cells, by establishing an electrophysiological measuring configuration in which a cell membrane forms a high resistive seal around a measuring electrode, making it possible to determine and monitor a current flow through the cell membrane. The substrate is typically part of an apparatus for studying electrical events in cell membranes, such as an apparatus for carrying out patch clamp techniques utilised to study ion transfer channels in biological membranes. More particularly, the invention relates to a substrate for such patch clamp apparatus having high through-put and utilising only small amounts of test compounds, only small amounts of liquid carrier, and being capable of carrying out many tests in a short period of time by performing parallel tests on a number of cells simultaneously and independently.

### BACKGROUND ART

The general idea of electrically insulating a patch of membrane and studying the ion channels in that patch under voltage-clamp conditions was outlined by Neher, Sakmann, and Steinback in "The Extracellular Patch Clamp, A Method For Resolving Currents Through Individual Open Channels In Biological Membranes", Pflueger Arch. 375; 219-278, 1978. They found that, by pressing a pipette containing acetylcholine (ACh) against the surface of a muscle cell membrane, they could see discrete jumps in electrical current that were attributable to the opening and closing of ACh-activated ion channels. However, they were limited in their work by the fact that the resistance of the seal between the glass of the pipette and the membrane (10-50 MΩ) was very small relative to the resistance of the channel (10 GΩ). The electrical noise resulting from such a seal is inversely related to the resistance and was large enough to obscure the currents flowing through ion channels, the conductance of which are smaller than that of the ACh channel. It also prohibited the clamping of the voltage in the pipette to values different from that of the bath due to the large currents through the seal that would result.

It was then discovered that by fire polishing the glass pipettes and by applying suction to the interior of the pipette a seal of very high resistance (1-100 GΩ) could be obtained with the surface of the cell. This Giga-seal reduced the noise by an order of magnitude to levels at which most channels of biological interest can be studied and greatly extended the voltage range over which these studies could be made. This improved seal has been termed a "giga-seal", and the pipette has been termed a "patch pipette". A more detailed description of the giga-seal may be found in O.P. Hamill, A. Marty, E. Neher, B. Sakmann & F.J. Sigworth: Improved patch-clamp techniques for high resolution current recordings from cells and cell-free membrane patches. Pflügers Arch. 391, 85-100, 1981. For their work in developing the patch clamp technique, Neher and Sakmann were awarded the 1991 Nobel Prize in Physiology and Medicine.

Ion channels are transmembrane proteins which catalyse transport of inorganic ions across cell membranes. The ion channels participate in processes as diverse as generating and timing action potentials, synaptic transmission, secretion of hormones, contraction of muscles, etc. Many drugs exert their specific effects via modulation of ion channels. Examples are antiepileptic compounds like phenytoin and lamotrigine which block voltage-dependent Na⁺-channels in the brain, antihypertensive drugs like nifedipine and diltiazem which block voltage dependent Ca²⁺-channels in smooth muscle cells, and stimulators of insulin release like glibenclamide and tolbutamide which block an ATP-regulated K⁺-channel in the pancreas. In addition to chemically induced modulation of ion-channel activity, the patch clamp technique has enabled scientists to perform manipulations with voltage dependent channels. These techniques include adjusting the polarity of the electrode in the patch pipette and altering the saline composition to moderate the free ion levels in the bath solution.

The patch clamp technique represents a major development in biology and medicine, since this technique allows measurement of ion flow through single ion channel proteins, and also allows the study of the single ion channel responses to drugs. Briefly, in standard patch clamp technique, a thin (app. 0.5-2 µm in diameter) glass pipette is used. The tip of this patch pipette is pressed against the surface of the cell membrane. The pipette tip seals tightly to the cell and isolates a few ion channel proteins in a tiny patch of membrane.

The activity of these channels can be measured individually (single channel recording) or, alternatively, the patch can be ruptured allowing measurements of the channel activity of the entire cell membrane (whole cell recording). High-conductance access to the cell interior for performing measurements can be obtained, e.g., by rupturing the membrane by applying subatmospheric pressure in the pipette.

During both single channel recording and whole-cell recording, the activity of individual channel subtypes can be characterised by imposing a "voltage clamp" across the membrane. In the voltage clamp technique the membrane current is recorded at a constant membrane potential. Or - to be more precise - the amplifier supplies exactly the current, which is necessary to keep the membrane potential at a level determined by the experimenter. Hence, currents resulting from opening and closing of ion channels are not allowed to recharge the membrane.

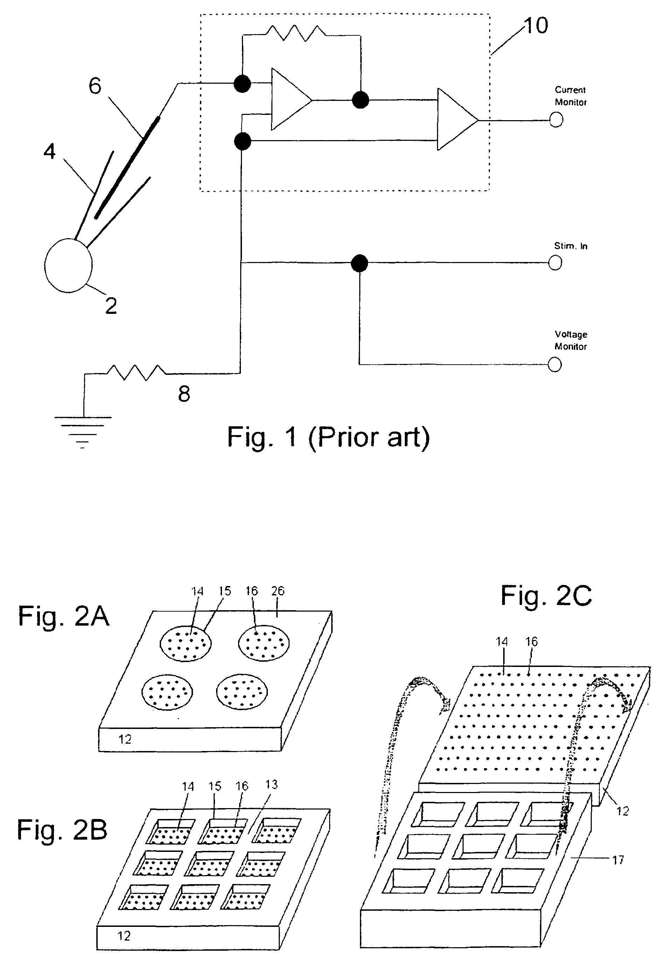

Figure 1 shows a simplified diagram of the basic operation of a standard prior art voltage clamp amplifier such as the EPC-9 amplifier from HEKA Elektronik. An electrode 6 inside a pipette 4 is connected to the negative terminal of a feedback amplifier, while the clamping voltage (referred to a grounded bath electrode (8)) is connected to a positive terminal (from Stim. In.) and made available at a voltage monitor output. Since the measured pipette voltage and the clamp voltage are supposed to be identical, a correction potential is constantly supplied at the pipette electrode as a current forced through the large feedback resistor. After inversion, the current is made available as an analogue voltage at the Current Monitor output.

The time resolution and voltage control in such experiments are impressive, often in the msec or even µsec range. However, a major obstacle of the patch clamp technique as a general method in pharmacological screening has been the limited number of compounds that could be tested per day (typically no more than 1 or 2). Also, the very slow rate of solution change that can be accomplished around cells and patches may constitute a major obstacle.

A major limitation determining the throughput of the patch clamp technique is localisation and clamping of cells and pipette, and the nature of the feeding system, which leads the dissolved compound to cells and patches.

In usual patch clamp setups, cells are placed in experimental chambers which are continuously perfused with a physiological salt solution. The establishment of the cell-pipette connection in these chambers is time-consuming and troublesome. Compounds are applied by changing the inlet to a valve connected to a small number of feeding bottles. The required volumes of the supporting liquid and the sample to be tested are high.

High throughput systems for performing patch clamp measurements have been proposed, which typically consist of a substrate with a plurality of sites adapted to hold cells in a measuring configuration where the electrical properties of the cell membrane can be determined.

US 5,187,096, Rensselaer, discloses an apparatus for monitoring cell-substrate impedance of cells. Cells are cultured directly on the electrodes which are then covered with a plurality of cells, thus, measurements on individual cells can not be performed.

WO 98/54294, Leland Stanford, discloses a substrate with wells containing electrode arrays. The substrate with wells and electrodes (metal electrodes) is made of silicon using CVD (Chemical Vapor Deposition) and etching techniques and comprises Silicon Nitride "passivation" layers surrounding the electrodes. The cells are cultivated directly on the electrode array. The substrate is adapted to measure electrophysiological properties and discloses a variety of proposed measuring schemes.

WO 99/66329, Cenes, discloses a substrate with perforations arranged in wells and electrodes provided on each side of the substrate. The substrate is made by perforating a silicon substrate with a laser and may be coated with anti-adhesive material on the surface. The substrate is adapted to establish giga seals with cells by positioning the cells on the perforations using suction creating a liquid flow through the perforations, providing the anti-adhesion layer surrounding the perforations, or by guiding the cells electrically. The cells can be permeabilised by EM fields or chemical methods in order to provide a whole-cell measuring configuration. All perforations, and hence all measurable cells, in a well share one working electrode and one reference electrode, see Figure 1, hence measurements on individual cells can not be performed.

WO 99/31503, Vogel et al., discloses a measuring device with an aperture arranged in a well on a substrate (carrier) and separating two compartments. The measuring device comprises two electrodes positioned on either side of the aperture and adapted to position a cell at the aperture opening. The substrate may have hydrophobic and hydrophilic regions in order to guide the positioning of the cells at the aperture opening.

WO 00/73784 (priority date 28.05.1999, publication date 07.12.2000), Case Western Reserve University, discloses a method of measuring efflux/influx of chemical drug, such as doxorubicin from a cell, such as a human cancer cell or other biological cell. The method includes introducing the drug to the cell, for example by immersing the cell in a culture medium containing the drug or by direct injection into the cell. The cell containing the drug is then immersed in a drug-free liquid medium and the efflux/influx of the drug from the cell measured with electrochemical equipment. For detection of efflux from small populations of cells, or a single cell, the signal strength may be increased by preconcentrating the drug on a working electrode and by increasing the oxygen concentration of the medium around the cell. A cell or small group of cells to be studied may be spatially positioned on a substrate by coating a small area of the substrate with a layer of hydrophilic material to which the cell attaches.

### SUMMARY OF THE INVENTION

The present invention provides a substrate and a method optimised for determining or monitoring current flow through ion channel-containing structures such as cell membranes, with a high throughput and reliability and under conditions that are realistic with respect to the influences to which the cells or cell membranes are subjected. Thus, the results determined using the substrate and the method of the invention, e.g., variations in ion channel activity as a result of influencing the cell membrane with, e.g., various test compounds, can be relied upon as true manifestations of the influences proper and not of artefacts introduced by the measuring system, and can be used as a valid basis for studying electrophysiological phenomena related to the conductivity or capacitance of cell membranes under given conditions.

This is because the current through one or more ion channels is directly measured using reversible electrodes as characterized below, typically silver/silver halide electrodes such as silver chloride electrodes, as both measuring electrodes and reference electrodes.

The substrate and method of the invention may be used not only for measurements on cell membranes, but also on other ion channel-containing structures, such as artificial membranes. The invention permits performing several tests, such as electrophysilogical measurements on ion transfer channels and membranes, simultaneously and independently. The substrate of the invention constitutes a complete and easily handled microsystem which uses only small amounts of supporting liquid (a physiological salt solution, isotonic with the cells, that is, normally having an osmolarity of 150 millimolar NaCl or another suitable salt) and small amounts of test samples.

In one aspect, the invention relates to an assembly comprising:
a plane substrate having a first surface part and an opposite second surface part, the first surface part having a plurality of sites each of which is adapted to hold an ion channel-containing structure contained in a liquid, and each of which has a passage therein through the substrate, the passage connecting the first surface part and the second surface part, which passage has walls and is dimensioned to hold an ion channel-containing structure,
a plurality of measuring electrodes, each of which is associated with a respective site,
one or more reference electrodes, the measuring electrodes and the respective reference electrode or reference electrodes being electrodes capable of passing, when in electrolytic contact with each other and when a potential difference is applied between them, a current between them by delivery of ions by one electrode and receipt of ions by the other electrode,

characterised in that:
each of the sites is adapted to provide a high electrical resistance seal established between an area of contact of the outer surface of an ion channel-containing structure held at the site, and the first surface part of the substrate around, or along the walls of said passage, the seal, when established, separating a domain defined on one side of the seal established by the ion channel-containing structure and in electrolytic contact with the measuring electrode, from a domain defined on the opposite side of the seal established by the ion channel-containing structure and in electrolytic contact with the respective reference electrode, whereby a current flowing between the reference and respective measuring electrodes and through the ion channel-containing structure can be determined and/or monitored,
the electrodes being integrated with the assembly, wherein the assembly further comprises a plurality of flow channel structures created in the substrate for delivering liquid to the plurality of sites.

In another aspect, the invention relates to a method of establishing a whole cell measuring configuration for determining and/or monitoring an electrophysiological property of one or more ion channels of one or more ion channel-containing structures, said method comprising the steps of:
providing an assembly comprising: a plane substrate having a first surface part and an opposite second surface part, said substrate having a plurality of sites in the first surface part of the substrate, each of which is adapted to hold an ion channel-containing structure and each of which has a passage therein through the substrate connecting the first surface part and the second surface part, which passage has walls and is dimensioned to hold an ion channel-containing structure and to form a high resistance seal between said ion channel-containing structure and said substrate, around or along the walls of said passage, a plurality of measuring electrodes, each of which is associated with a respective site, and one or more reference electrodes;
supplying a carrier liquid at one or more sites using a plurality of flow channel structures created in the substrate, said carrier liquid containing one or more ion channel-containing structures the carrier liquid contacting the first surface part of the substrate;
positioning at least one of the ion channel-containing structures at a corresponding number of sites;
forming a high electrical resistance seal between an area of contact of the outer surface of an ion channel-containing structure held at the site and a first surface part of the substrate around or along the walls of said passage, the seal, when established, separating a domain defined on one side of the seal established by the ion channel-containing structure and in electrolytic contact with the measuring electrode from a domain defined on the opposite side of the seal established by the ion channel-containing structure and in electrolytic contact with the respective reference electrode;
checking for a high electrical resistance seal between an ion channel-containing structure held at a site in the first surface part of the substrate or along the walls of said passage with which the high electrical resistance seal is to be established by successively applying a first electric potential difference between the measuring electrode associated with the site and a reference electrode, monitoring a first current flowing between said measuring electrode and said reference electrode, and comparing said first current to a predetermined threshold current and, if the first current is at most the predetermined threshold current, then approving the site as having an acceptable seal between the ion channel-containing structure and the first surface part of the site; and
establishing a whole-cell configuration at approved sites,
whereby a third current flowing through ion channels of the ion channel-containing structure between the measuring electrode and the reference electrodes can be determined and/or monitored.

An ion channel-containing structure in a solution may be guided towards a site on a substrate either by active or passive means. When the ion channel-containing structure makes contact with the site, e.g. substrate around an electrode, the contact surfaces form a high electrical resistance seal (a giga-seal) at the site, e.g. surrounding the electrode, so that an electrophysiological property of the ion channels can be measured using the respective electrode. Such electrophysiological property may be current conducted through the part of membrane of the ion channel-containing structure that is encircled by the giga-seal.

In the present context, the term "giga-seal" normally indicates a seal of a least 1G ohm, and this is the size of seal normally aimed at as a minimum, but for certain types of measurements where the currents are large, lower values may be sufficient as threshold values.

The whole-cell configuration may be obtained by applying, between the measuring electrode associated with each approved site and a reference electrode, a series of second electric potential difference pulses, monitoring a second current flowing between the measuring electrode and the reference electrode, and interrupting the series of second electric potential difference pulses whenever said second current exceeds a predetermined threshold value, thereby rupturing the part of the ion channel-containing structure which is closest to the measuring electrode.

Alternativelly, the whole-cell configuration may be obtained by subjecting the part of the ion channel-containing structure which is closest to the measuring electrode to interaction with a pore forming substance.

It should be noted that in the present context, the term "whole-cell configuration" denotes not only configurations in which a whole cell has been brought in contact with the substrate at a measuring site and has been punctured or, by means of a pore-forming substance, has been opened to electrical contact with the cell interior, but also configurations in which an excised cell membrane patch has been arranged so that the outer face of the membrane faces "upwardly", towards a test sample to be applied.

As the measuring electrode associated with a site is one of a plurality of electrodes on the substrate, and the ion channel-containing structure is one of many in a solution, it is possible to obtain many such prepared measuring set-ups on a substrate. A typical measurement comprises adding a specific test sample to the set-up, for which reason each measuring set-up is separated from other measuring set-ups to avoid mixing of test samples and electrical conduction in between set-ups.

### BRIEF DESCRIPTION OF THE DRAWINGS

The present invention will now be described in greater detail with reference to the accompanying drawings, in which:
Fg. 1, as mentioned above, shows a diagram of a typical known electronic circuit for voltage clamp measurements;
Fig. 2 shows a schematic view of examples of substrates having sites with electrodes for holding cell membranes or artificial membranes;
Fig. 3A-3D shows cross-sectional side views of substrates showing the different layers produced in wafer processing technology (deposition/photolithography/etching technology);
Fig. 4A shows a cross-sectional side view of another design for a substrate having sites with electrodes for holding cell membranes or artificial membranes:
Fig. 4B shows a top view of the structure of Figure 4A;
Fig. 5 shows a close-up of sites enclosed by a region of hydrophobic material;
Fig. 6 shows a test confinement with an array of electrodes connected to a line of contacts; and
Fg. 7 shows a flow diagram of a procedure for detecting when a cell forms a giga-seal with an substrate, e.g. around an electrode.

The reference numbers in the drawings refer to the following:

| No. | Description |
|---|---|
| 2 | cell |
| 4 | pipette |
| 6 | pipette measuring electrode |
| 8 | reference electrode |
| 10 | voltage clamp amplifier |
| 11 | edge of hydrophobic region |
| 12 | substrate |
| 13 | substructure |
| 14 | site |
| 15 | test confinement |
| 16 | electrode |
| 17 | second structure part |
| 18 | lines of conducting material |
| 20 | contacts |
| 22 | insulating film |
| 24 | Silver |
| 26 | hydrophobic region |
| 28 | AgCl layer |
| 30 | aperture |
| 31 | SiO₂ layer |
| 32 | piping |

### DESCRIPTION OF PREFERRED EMBODIMENTS

The present invention relates to a substrate with a plurality of electrodes at sites adapted to hold cells (or other ion channel-containing structures), such that the cell membrane and the substrate interface creates a giga-seal around an electrode, making it possible to determine or monitor electrophysiological properties of the cell membrane. It will be understood that when the term "cell" or "cell membrane" is used in the present specification, it will normally, depending on the context, be possible to use any other ion channel-containing structure, such as another ion channel-containing lipid membrane or an ion channel-containing artificial membrane. Electrophysiological properties can be, e.g., current flow through an ion channel or capacitance of an ion channel-containing membrane. It is possible to add individual test samples (typically pharmacological drugs) at each cell-holding location so that individual experiments can be carried out on each cell. An experiment can be to measure the response of the ion transfer channel to the addition of test sample. In order to carry out individual experiments, different test samples could be added to different cell-holding sites. One or more cell holding sites where a specific test sample is (going to be) added is hereafter called a test confinement.

The substrate of the invention will typically be a component used in an apparatus for carrying out measurements of the electrophysiological properties of ion transfer channels in lipid membranes such as cells.

The apparatus will be designed to provide means for carrying out a large number of individual experiments in a short period of time. This is accomplished by providing a microsystem having a plurality of test confinements each of which having sites comprising integrated measuring electrodes, and providing a suitable test sample supply. Each test confinement may comprise means for positioning cells, for establishment of giga-seal, for selection of sites at which giga-seal has been established, measuring electrodes and one or more reference electrodes. Thereby it is possible to perform independent experiments in each test confinement, and to control the preparation and measurements of all experiments from a central control unit such as a computer. Due to the small size of the test confinements, the invention permits carrying out measurements utilising only small amounts of supporting liquid and test sample. The present invention also provides several different procedures for carrying out measurements; these include measurements on fragments of cells and artificial membranes.

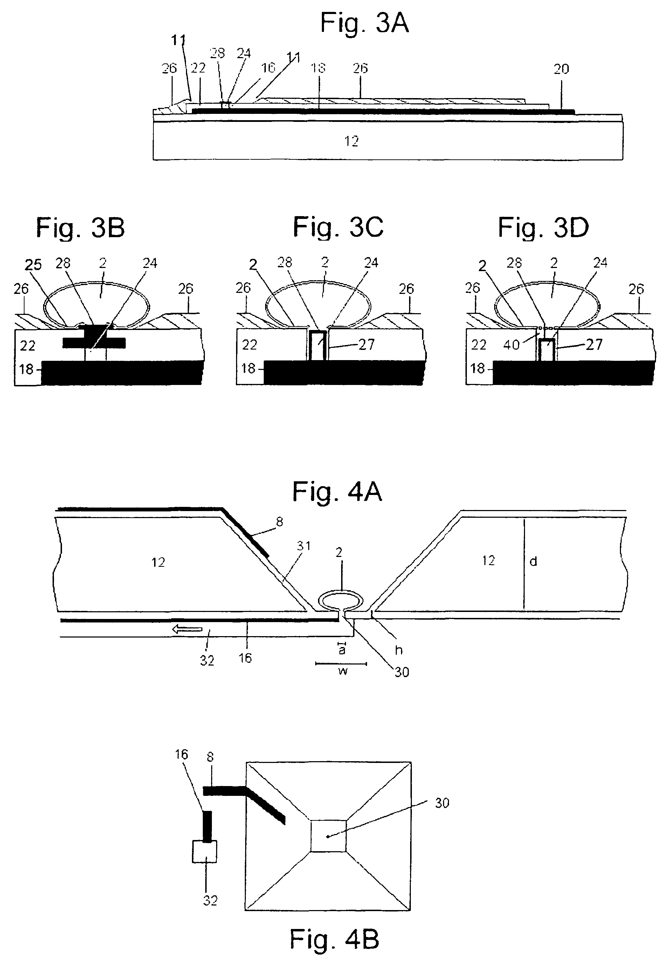

The substrate having sites with measuring electrodes (electrodes hereafter) can be designed in a number of ways, of which three are illustrated in Figures 2A-2C, and further ones are illustrated in Figures 4A-4B. The distinction between the embodiments is the design of the sites on the substrate. Sites are adapted to hold an ion channel-containing structure, such as a cell, in that the surface material at the site is well suited for creating a seal with the cell (or structure) membrane as described in the prior art. Such materials include silicon, plastics, pure silica and other glasses such as quartz and pyrex or silica doped with one or more dopants selected from the group of Be, Mg, Ca, B, Al, Ga, Ge, N, P, As and oxides from any of these. The substrate proper can be made of any material suitable for a wafer processing technology, such as silicon, plastics, pure silica and other glasses such as quartzs and pyrex or silica doped with one or more dopants selected from the group of Be, Mg, Ca, B, Al, Ga, Ge, N, P, As. Silicon is the presently preferred substrate material.

In the designs of Figures 2A-2C, the sites 14 are arranged on a locally flat surface of the substrate 12. Locally flat indicates that the surface of the substrate may have some substructure 13 on a scale larger than one or more sites, as seen in Figure 2B. Sites, and thereby electrodes 16, can be arranged alone or in groups within this substructure.

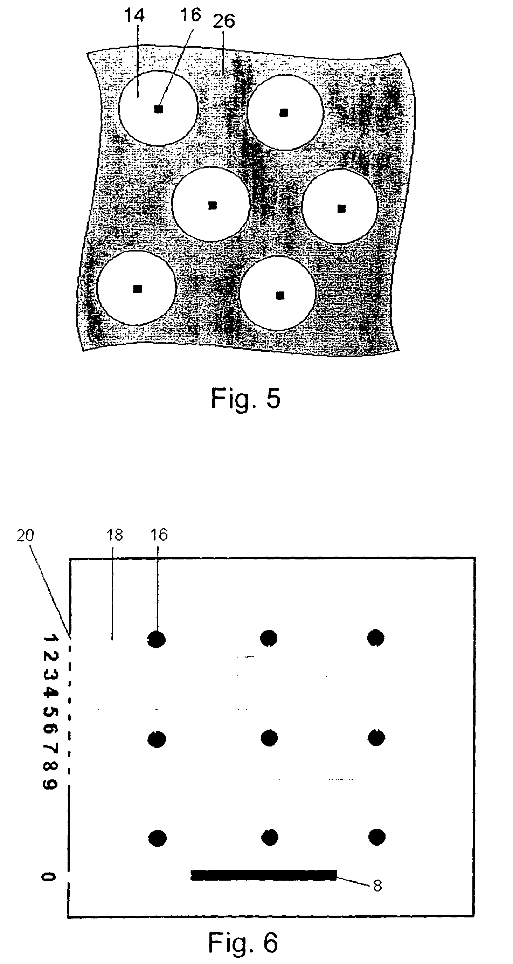

The methods for production of the three designs of Figure 2 are analogous to each other. Figure 2A and 2B simply includes some subdivision of the basic design of Figure 2C. The manufacture of the designs is now described with reference to Figures 3A and 6.

Lines 18 of conducting material are formed on the surface of the substrate by first depositing a layer of conducting material on the substrate. Deposition of materials on the substrate, and on other surfaces throughout the description, can be made using one of several deposition techniques, such as Physical Vapour Deposition which includes 1) application of material from a vapour phase, 2) spottering and 3) laser ablation; Chemical Vapour Deposition techniques which include 1) atmospheric pressure chemical vapour deposition (APCVD), 2) low pressure chemical vapour deposition (LPCVD), 3) plasma enhanced chemical vapour deposition (PECVD) and 4) photo enhanced chemical vapour deposition; as well as spin coating and growth techniques. Secondly, the individual wires are defined in a photolithography step, and thirdly, conducting material not being a part of the wires is removed by etching. The wires are preferably defined so that one part of the wires forms a line of contact pads 20 whereas another part forms an array of measuring electrode parts 16 and one or more reference electrodes 8. The array of electrode parts is not necessarily an ordered pattern. The contact pad and electrode part are preferably the two end parts of the wire, but may be any parts of e.g. a pattern of conducting strips. Preferably, the conducting material consists of metals or doped silicon.

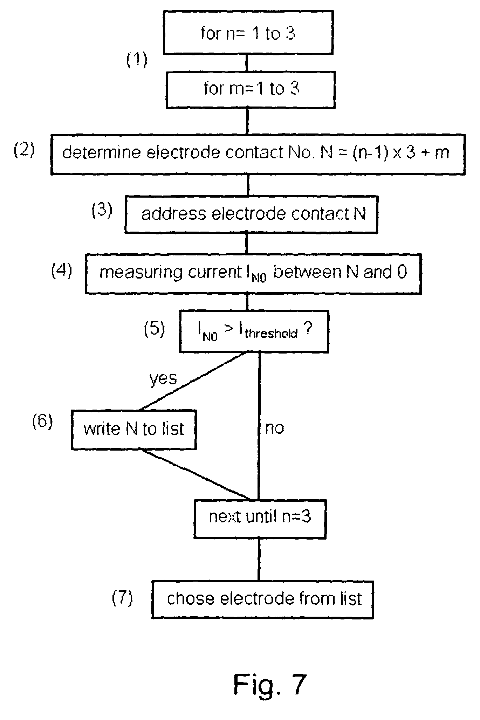

In order to establish the electrodes and contacts, the conducting material not forming part of the electrode or of the contact part of each wire is covered with an insulating (hydrophilic) film 22, e.g. silicon dioxide, or multiple layers of silicon nitride and silicon dioxide. This is carried out by covering the whole surface with a layer of the insulating film using either thermal oxidation of silicon, physical or chemical vapour deposition, or spin coating. Using photolithography and an etching step, parts of the insulating film are removed to expose the wire and thereby form electrodes 16 and 8 and contacts 20. For a better electrical contact, electrodes (and contacts) can be covered with silver 24. Alternatively, lift-off techniques might be used in these cases where several layers of material are to be deposited in several thin layers. Here a photoresist is deposited over the substrate and the pattern to be formed is defined in the resist by illumination through a mask followed by etching. A layer of material, typically a metal, is vapour deposited onto the structure, and the photoresist is dissolved, thereby leaving metal in the defined pattern. At this stage, the substrate will appear as shown in Figure 7, the thin lines 18 connecting electrodes and contacts being covered by insulating film.

Optionally, but shown in Figure 3A, hydrophobic regions 26 completely surrounding electrode sites or groups of sites are formed using a combination of deposition of a hydrophobic material like Teflon and photolithography. The hydrophobic material is deposited using either spin coating, chemical vapour deposition or plasma enhanced chemical vapour deposition. Figure 2A shows a possible use of such regions.

Finally, before use, a silver chloride layer 28 is formed on the electrode 16 using electrolytic treatment. The same procedure is normally followed for all measuring and reference electrodes in the substrates of the invention to establish them as silver/silver halide electrodes, such as silver/silver chloride electrodes.

Figure 3B shows a close-up of a site holding a cell 2 where the seal 25 is formed at the site surrounding the AgCl layer 28. In the production of the electrode, a large volume of AgCl layer 28 is formed on top of the silver 24 prior to deposition of the silica layer 22, thereby ensuring a large supply of AgCl.

Figure 3C shows an assembly wherein the measuring electrode is positioned in a small well 27 whereby the seal is formed between the membrane and the rim of the well 27. Depending on the size of the well 27, this assembly allows for a greater separation of the membrane and the working electrode as well as for a larger volume of the carrier liquid surrounding the electrode.

Figure 3D shows another assembly wherein the working electrode is positioned in a small well 27 as in Figure 3C. Here, a pore-forming substance 40 has been deposited at the site in order to establish, by the action of dissolved pore-forming substance on the cell, a whole-cell measuring configuration when a cell is positioned.

In the design of Figure 4A, a site is positioned at the bottom of a well, a geometrically shaped structure on the substrate. The function of the well is both to position the cell 2 at the site and to separate test confinements, which in this case consist of single sites.

A substrate with a well shaped as a truncated pyramid is shown in Figure 4A, an aperture or passage 30 from the narrow end of the truncated pyramid to the bottom surface part of the substrate is also defined in the substrate, the well and the passage thereby creating a funnel. A measuring electrode 16 is provided on the bottom surface part of the substrate close to the aperture or passage, and a reference electrode 8 is provided at a side surfaces of the well, as shown in Figure 4A. Preferably there is provided piping 32 for applying suction to the passage on the bottom side of the substrate. In a preferred embodiment, this piping leads to the upper side of the substrate, and may include the electrical wiring to the measuring electrode.

When the term "bottom" is applied above, this merely refers to the orientation of the drawing. In the use of the substrate according to the invention, it is not a condition that the first surface part of the substrate is the upper surface part and the second surface part the lower surface part. In other words, gravity is not utilized to any substantial extent in connection with these very small structures, and, as an example, the design of Fig. 4A could also be used in an orientation corresponding to the figure having been rotated an angle of 180 degrees (or any other angle, for that matter).

The well shown in Figure 4A is basically a truncated pyramidal cavity with a hole 30 at the apex. The base of the pyramid is a square. The top angle of the pyramid is 2x54.7°, the wafer thickness *d* = 350-650µm, the side-length at the apex of the pyramid is *w* ≈ 30µm in order to allow room for a cell. The apex of the pyramid is covered with a Silicon-dioxide membrane 31 of thickness *h* ≈ 3µm. In this membrane, a hole of diameter *a* ≈ 0.1-10 µm, such as 1-5µm, is formed.

The structure comprising a well or wells can be made in several quite different ways. Below, two different production processes for the basic structure are summarised, the *oxide first* process and the oxide *last* process, respectively

### Oxide first process

- Grow 3µm wet thermal SiO₂ covering whole substrate.
- Define the hole on the bottom side of the substrate by photomasking and Reactive Ion Etching to make the hole through the oxide to the silicon substrate.
- Deposit LPCVD Silicon-nitride for an etch mask on both sides of the substrate.
- Define nitride windows to form pyramid base plane on the upper side of the substrate by photomasking and Reactive Ion Etching and wet oxide etching (buffered Fluoric Acid)
- Etch pyramidal cavities through the windows by anisotropic etching in the silicon. This creates pyramid sides with a slope of 54,7°.
- Strip nitride etch stop using hot H₃PO₄.
- Grow 1 µm wet thermal SiO₂ to electrically insulate the bulk silicon wafer in order to cover the sides of the pyramid. Other SiO₂ regions will not grow considerably.

### Oxide last process

- Form an etch-stop layer in silicon (boron doping) on the bottom side of the substrate, using either doping by implantation or epitaxial growth. The etch stop layer will typically be around 1 µm thick.
- Deposit LPCVD silicon nitride for an etch mask on both sides of the substrate.
- Define nitride windows to form pyramid base plane on the upper side of the substrate by photomasking and Reactive Ion Etching and wet oxide etching (buffered Fluoric Acid)
- Etch pyramidal cavities through the windows by anisotropic etching in the silicon. This creates pyramid sides with a slope of 54,7°. The etching stops on the boron-doped etch stop to form an ∼ 1 µm thick silicon membrane.
- Strip nitride etch stop using Hot H₃PO₄.
- Define the hole on the bottom side by photomasking and Reactive Ion Etching of Silicon
- Grow wet thermal SiO₂ to convert the Silicon membrane into an oxide everywhere on the substrate. This process shrink the hole since SiO₂ is also formed inside the hole, which thereby can be made smaller compared to what is possible using photolithography.

For both production processes the main concern during processing is the mechanical stability of the SiO₂ membrane with the hole during the final high temperature oxidation step. The surface material (here SiO₂) can optionally be coated with silicon nitride, in order to prevent a contribution to the electrical conductivity.

Measuring and reference electrodes can now be formed. The measuring electrode on the bottom side can be formed using standard deposition and photolithography techniques. The reference electrode is preferably formed using evaporation of conducting material through a shadow mask, or through use of an electrophoretic resist technique.

Further, flow channel structures for adding liquid to the funnel may possibly be created in the substrate, giving an in-flow and an out-flow port to/from the funnel and elsewhere on the substrate. Alternatively, the flow channels are made on another substrate to be applied on top of the substrate, using normal etching techniques.

The features described are preferably arranged such that there is an easy access to all connection in- and outlets from above the assembly, as illustrated in Figure 4B (suction outlet 32, contacts to measuring electrode 16 and reference electrode 8). This preferred configuration is adapted for applying a unit, having similar but reverse in- and outlets, on top of the assembly.

It is an important aspect that the substrate can provide some means for separating test confinements 15 as in Figure 2. Test confinements preferably hold volumes as small as nanolitres. This is convenient considering the necessary amounts of the often expensive test samples; moreover, the time needed for mixing the solution by diffusion decreases with decreasing volume.

In Figure 2A, the test confinements are defined using surface materials to define hydrophobic regions 26 and hydrophilic sites 14 on the substrate, as described previously. If the surface is wetted (but not flooded) by an aqueous solution such as saline, the liquid will confine itself to the hydrophilic areas, thereby defining the test confinements. Each hydrophilic area includes some sites 14 with electrodes 16 and may also include smaller scale hydrophobic areas.

On the substrate shown in Figure 2B, the test confinements are separated by subdivisions 13 formed on the surface of the substrate. These subdivisions can be produced on the raw substrate by covering the substrate surface with a resist, and define the well openings using photolithography. An etch step followed by removal of the remaining resist leaves the substrate ready for formation of sites and electrodes.

Figure 2C shows a substrate covered with electrodes, without any substantial subdivision. In this case the test confinements are defined using a structure part 17 with hollow subdivisions/chambers, to be applied on top of the substrate. By making a tight mechanical contact with the substrate, the structure part forms closed chambers each holding one or more sites with electrodes. If convenient, a similar structure part can be applied on top of any of the substrates shown in Figure 2A and B.

In all of the embodiments shown in Figure 2, a reference electrode has to be located within each test confinement. This can be realised either by having an electrode at a site where no cell can cover it, an electrode so large that no cell can cover it, or, by dosing the number of cells in such a way that cells can not cover all electrodes. This last option allows for any of the measuring electrodes to function as reference electrode.

Depending on the specific shape of the substrate with electrodes, the addition of cell-supporting liquid and cells is carried out in one of the following ways. In a preferred embodiment, the test confinements are accessible from above, and droplets of supporting liquid and cells can be supplied at each test confinement by means of a dispensing or pipetting system. Systems such as an ink jet printer head or a bubble jet printer head can be used. Another possibility is an nQUAD aspirate dispenser or any other dispensing/pipetting device adapted to dose small amounts of liquid. Alternatively, supporting liquid and cells are applied on the substrate as a whole (e.g. by pouring supporting liquid containing cells over the substrate or immersing the substrate in such), thereby providing supporting liquid and cells to each test confinement. Since the volumes of supporting liquid and later test samples are as small as nanolitres, water vaporisation could represent a problem. Therefore, depending of the specific volumes, handling of liquids on the substrate should preferably be carried out in high humidity atmospheres.

In the case of the test confinements being closed chambers, they might only be accessible through a system of channels, i.e. a microliquid handling system. This is the case when a second structure part 17 (Figure 2C) is applied on top of any of the substrates with or without test confinements. In this case supporting liquid and cells must be provided through inlet channels typically defined in the second structure part 17. Such a second structure part can be made of, e.g. silicon in which case flow channels can be formed using standard photolithography and etching techniques. Such a second structure part can be applied on top of any of the embodiments.

In another aspect, the cells are cultivated directly on the substrate, while immersed in growth medium. In the optimal case, the cells will form a homogeneous monolayer (depending on the type of cells to be grown) on the entire surface, except at regions where the surface intentionally is made unsuitable for cell growth. The success of cultivation of cells on the substrate depends strongly on the substrate material.

In still another aspect, an artificial membrane with incorporated ion channels may be used instead of a cell. Such artificial membrane can be made from a saturated solution of lipids, by positioning a small lump of lipid over an aperture. This technique is thoroughly described in e.g. "Ion Channel Reconstitution" by Christopher Miller, *Plenum* 1986, p. 577. If the aperture size is appropriate, and a polar liquid such as water is present on both sides of the aperture, a lipid bilayer can form over the aperture. The next step is to incorporate a protein ion channel into the bilayer. This can be achieved by supplying lipid vesicles with incorporated ion channels on one side of the bilayer. The vesicles can be drawn to fusion with the bilayer by e.g. osmotic gradients, whereby the ion channels are incorporated into the bilayer.

Obtaining good contact between the cell and a glass pipette, and thereby creating a giga-seal between a cell and the tip the pipette, is well described in the prior art. In order to draw the cell to the tip of the pipette, as well as to make the necessary contact for obtaining the giga-seal, it is normal to apply suction to the pipette.

In the case of the substrates described in Figures 2A - C, no suction is provided, and the positioning of the cells is carried out by other means. Moreover, it has been shown that the mere contact between the cell membrane and the substrate, typically ultra-pure silica, is sufficient for the cell to make some bonding to the surface and create a giga-seal.

The positioning can be carried out by electrophoresis, where an electric field from an electrode draws the charged cell towards it. Negatively charged cells will be drawn towards positive electrodes and vice versa. The electrostatic pull can also act as guiding means for a group of electrodes. Alternatively, within a test confinement, a hydrophobic material 26 may cover the surface of the substrate except at areas just around electrodes. This is shown in Figure 5. Thereby, cells can only bind themselves on electrode sites 14. It is possible to apply both of these methods simultaneously or optionally in combination with a suitable geometrical shape of the substrate surface around electrodes, to guide the sinking cells towards the electrode.

In another embodiment, the density and pattern of sites and measuring electrodes is close to or higher than the density of cells when these are packed to make closest packing on the surface of the substrate. This ensures that when a sufficient number of cells is supplied, at least one electrode is covered by a cell without further guiding means.

In the embodiment shown in Figure 4A, one or more cells 2 in a supporting liquid are applied and sink to the bottom end of the funnel, this being an example of positioning by geometrical shaping. If suction is applied, it draws the cell to the aperture 30 and establishes a connection between the cell and the aperture, creating a giga-seal separating the aperture inside and the solution. The giga-seal may take any form, e.g., circular, oval or rectangular. The supporting liquid makes electrical contact between the cell membrane and the reference electrode. The cell may be deformed by the suction, and a case where the cell extends into the aperture may be desired if controlled.

Each test confinement preferably holds several electrode sites. In order to detect whether an electrode is covered by a cell and insulated by a giga-seal, leak currents are measured between electrodes or between electrodes and the reference electrode. Even though a test confinement may include numerous electrodes, it is a simple task to search for electrodes insulated by giga-seals, a task well suited for a computer.

Figure 6 and 7 proposes a scheme for doing so, where the electrodes 16 in a test confinement form an n×m matrix (here 3x3). The electrode connections 18 lead to a line of contacts 20 (No. 1 to 9) on the substrate that can be individually addressed by a computer with means for measuring currents. A list of giga-sealed electrodes can be made using a simple method sketched in the flow diagram of Figure 7. First (1), two loops are established for going through all entries in the matrix of electrodes. In (2), the n×m array of the matrix is unfolded to provide an individual addressing (3) of electrode contacts with an electrode contact number N (No. 1 to 9). The current, at an applied voltage between contact N and the reference electrode 8, contact No. 0, is measured (4), and its value is compared to some threshold current I_{threshold} (5) for determining whether the electrode is giga-sealed. If a giga-seal is detected, the contact number is added to a list of suitable electrodes (6) from which a measuring electrode is selected (7). This scheme carries some information on the relative positions n,m of suitable electrodes. This information can be used for selecting the optimal measuring electrode in (7), but can be omitted so that each electrode is simply known by its contact number N. Typically, only one electrode per test confinement is chosen.

The activity of these channels can be measured electrically (single channel recording) or, alternatively, the patch can be ruptured allowing measurements of the channel activity of the entire cell membrane (whole cell recording). High-conductance access to the cell interior for performing whole cell measurements can be obtained in at least 3 different ways (all methods are feasible, but various cells may work better with different approaches):
a) In the embodiment shown in Figure 4A, the membrane can be ruptured by suction from the aperture side. Subatmospheric pressures are applied either as short pulses of increasing strength or as ramps or steps of increasing strength. Membrane rupture is detected by highly increased capacitative current spikes (reflecting the total cell membrane capacitance) in response to a given voltage test pulse.
b) Membrane rupture by applied voltage pulses. Voltage pulses are applied either as short pulses of increasing strength (mV to V) and duration (u- to msec), or as ramps or steps of increasing strength, between the electrodes. The lipids forming the membrane of a typical cell will be influenced by the large electrical field strength from the voltage pulses, whereby the membrane to disintegrates in the vicinity of the electrode. Membrane rupture is detected by highly increased capacitative current spikes in response to a given voltage test pulse.
c) Permeabilization of membrane. Application of pore-forming substances (for example antibiotics such as nystatin or amphotericin B), by e.g. prior deposition of these at the site. Rather than by rupturing the membrane, the membrane resistance is selectively lowered by incorporation of permeabilizing molecules, resulting in effective cell voltage control via the electrode pair. The incorporation is followed by a gradually decreasing total resistance and an increasing capacitance.

At this stage, a substrate with some electrodes each holding a cell is provided, the selected cells form a giga-seal around their respective electrodes, allowing for the electrode to measure electrophysiological properties of the ion transfer channels in the cell membrane. This represents the main aspect of the invention, the making available of a plurality of prepared sample cells for performing electro-physiological experiments. Moreover, each cell is confined in order to permit individual testing of the cells.

The remaining of this description will focus on the application of the substrate made ready in this way.

The test samples must be added to each test confinement individually, with different test samples for each test confinement. This can be carried out using the methods for applying supporting liquid, with the exception of the methods where supporting liquid are applied on the substrate as a whole.

Upon positioning the cell in a measuring configuration, several electrophysiological properties can be measured, such as current through ion channels (voltage clamp), or capacitance of ion channels containing membranes. In any case, a suitable electronic measuring circuit should be provided. The person skilled in the art will be able to select such suitable measuring circuit. One such possible circuit for voltage clamp measurements is described above with reference to Figure 1.

In the case of voltage clamp measurements, the electrical current carried by the ion transfer channels in the cell membrane results in a charge transfer from the solution (reference electrode) to the measuring electrode, typically of the order of pA to µA (picoampere - 10⁻¹²A). A low noise amplifier is provided for measuring these currents. The electronic circuits can be integrated in a separate standard unit having contact to the two electrodes and possibly flow channels for drug application.

## Claims

1. An assembly comprising:
a plane substrate (12) having a first surface part and an opposite second surface part, the first surface part having a plurality of sites (14) each of which is adapted to hold an ion channel-containing structure (2) contained in a liquid, and each of which has a passage (30) therein through the substrate, the passage connecting the first surface part and the second surface part, which passage has walls and is dimensioned to hold an ion channel-containing structure,
a plurality of measuring electrodes (16), each of which is associated with a respective site,
one or more reference electrodes (8), the measuring electrodes and the respective reference electrode or reference electrodes being electrodes capable of passing, when in electrolytic contact with each other and when a potential difference is applied between them, a current between them by delivery of ions by one electrode and receipt of ions by the other electrode,
**characterised in that**:
each of the sites (14) is adapted to provide a high electrical resistance seal established between an area of contact of the outer surface of an ion channel-containing structure held at the site, and the first surface part of the substrate around, or along the walls of said passage (30), the seal, when established, separating a domain defined on one side of the seal established by the ion channel-containing structure (2) and in electrolytic contact with the measuring electrode, from a domain defined on the opposite side of the seal established by the ion channel-containing structure and in electrolytic contact with the respective reference electrode (8), whereby a current flowing between the reference (8) and respective measuring (16) electrodes and through the ion channel-containing structure can be determined and/or monitored,
the electrodes (16,8) being integrated with the assembly, wherein the assembly further comprises a plurality of flow channel structures created in the substrate for delivering liquid to the plurality of sites.

2. The assembly according to Claim 1, further defining a piping (32) for the application of the suction, the piping comprising electrical wiring for the measuring electrode.

3. The assembly according to Claim 1 or Claim 2, wherein each passage (30) is connected to a suction providing means so as to enable subjecting an internal volume of the passage of a selected site to a suction generating a flow of carrier liquid through the passage for guiding ion channel-containing structures towards the passage.

4. The assembly according to any one of Claims 1 to 3, wherein the electrodes (16, 8) have been formed by a wafer processing technology.

5. The assembly according to any one of the preceding claims, wherein the substrate (12) is a silicon substrate, and the surface part of the site with which the high electrical resistance seal is to be established is a silica surface part, and the electrodes have been formed by a process comprising deposition/photolithography/etching process.

6. The assembly according to any one of the preceding claims, wherein the plurality of sites (14) is arranged in an array on the first surface part of the substrate.

7. The assembly according to Claim 6, wherein the array of sites (14) comprises at least 9 sites.

8. The assembly according to any one of the preceding claims, wherein the measuring (16) and reference (8) electrodes are silver/silver halide electrodes.

9. The assembly according to any one of Claims 1 to 7, wherein the measuring (16) and reference (8) electrodes are silver/silver chloride electrodes.

10. The assembly according to any one of the preceding claims, comprising a first layer of hydrophobic material (26) positioned on or above the surface of the substrate, said first layer covering only parts of the surface of the substrate.

11. The assembly according to Claim 10, where one or more sites (14) are located within parts of the surface of the substrate not covered by said first layer.

12. The assembly according to any one of the preceding claims, comprising one or more wells extending into the substrate (12) and having well openings defined in the first surface part, each having a bottom part and a side part, at least some of the sites (14) of the first surface part being positioned within the bottom parts of the wells for delivering liquid to said plurality of sites, such that a well and a passage together define a funnel.

13. The assembly according to Claim 12, wherein the plurality of flow channel structures enable liquid to be added to the one or more funnels.

14. The assembly according to Claim 13, wherein the plurality of flow channel structures comprises an inflow and out flow port to/from the one or more funnels.

15. The assembly according to any one of Claims 12 to 14, wherein the wells have been formed by a process comprising a photolithography/etching process.

16. The assembly according to Claim 15, wherein the substrate (12) is a silicon substrate, and wherein the wells are shaped as truncated pyramids the bottoms of which are constituted by the well openings and the side parts of which have a slope of 54.7°.

17. The assembly according to any one of the preceding claims, wherein the transverse dimension of the passage (30) is 1-5µm.

18. The assembly according to any of Claims 12 to 17, wherein a reference electrode (8) is positioned on the side part of each well.

19. The assembly according to any one of the preceding claims, wherein the measuring electrode (16) associated with each site is positioned at each respective site.

20. The assembly according to any one of Claims 1 to 18, wherein the measuring electrode (16) associated with each site (14) is positioned on the opposite second surface part of the substrate (12).

21. The assembly according to Claim 20, wherein the measuring electrode (16) associated with each site (14) is positioned adjacent to an opening of the passage defined at the respective site.

22. The assembly according to any one of the preceding claims, further comprising, for each of the sites (14), an electronic circuit that is connected with the respective measuring electrode (16) and with the reference electrode (8) or one of the reference electrodes for generation of an amplified signal that is a unique function of a current flowing through ion channels between said electrodes.

23. A method of establishing a whole cell measuring configuration for determining and/or monitoring an electrophysiological property of one or more ion channels of one or more ion channel-containing structures (2), said method comprising the steps of:
providing an assembly comprising: a plane substrate having a first surface part and an opposite second surface part, said substrate having a plurality of sites (14) in the first surface part of the substrate, each of which is adapted to hold an ion channel-containing structure and each of which has a passage (30) therein through the substrate connecting the first surface part and the second surface part, which passage has walls and is dimensioned to hold an ion channel-containing structure and to form a high resistance seal between said ion channel-containing structure and said substrate, around or along the walls of said passage, a plurality of measuring electrodes (16), each of which is associated with a respective site, and one or more reference electrodes (8);
supplying a carrier liquid at one or more sites using a plurality of flow channel structures created in the substrate, said carrier liquid containing one or more ion channel-containing structures the carrier liquid contacting the first surface part of the substrate;
positioning at least one of the ion channel-containing structures at a corresponding number of sites;
forming a high electrical resistance seal between an area of contact of the outer surface of an ion channel-containing structure held at the site and a first surface part of the substrate around or along the walls of said passage, the seal, when established, separating a domain defined on one side of the seal established by the ion channel-containing structure and in electrolytic contact with the measuring electrode from a domain defined on the opposite side of the seal established by the ion channel-containing structure and in electrolytic contact with the respective reference electrode;
checking for a high electrical resistance seal between an ion channel-containing structure (2) held at a site (14) in the first surface part of the substrate or along the walls of said passage with which the high electrical resistance seal is to be established by successively applying a first electric potential difference between the measuring electrode (16) associated with the site and a reference electrode (8), monitoring a first current flowing between said measuring electrode and said reference electrode, and comparing said first current to a predetermined threshold current and, if the first current is at most the predetermined threshold current, then approving the site as having an acceptable seal between the ion channel-containing structure and the first surface part of the site; and
establishing a whole-cell configuration at approved sites,
whereby a third current flowing through ion channels of the ion channel-containing structure between the measuring electrode and the reference electrodes can be determined and/or monitored.

24. The method according to Claim 23, wherein the step of establishing a whole-cell configuration at approved sites comprises applying, between the measuring electrode (16) associated with each approved site and a reference electrode (8), a series of second electric potential difference pulses, monitoring a second current flowing between the measuring electrode and the reference electrode, and interrupting the series of second electric potential difference pulses whenever said second current exceeds a predetermined threshold value, thereby rupturing the part of the ion channel-containing structure which is closest to the measuring electrode.

25. The method according to Claim 23 or Claim 24, wherein the step of establishing a whole-cell configuration at approved sites comprises subjecting the part of the ion channel-containing structure which is closest to the measuring electrode to interaction with a pore forming substance.

26. The method according to any one of Claims 23 to 25 and any claim dependent thereon, wherein the substrate defines, at each of the sites, a passage connecting the first and the second surface parts, the passage being positioned substantially in a middle part of the surface part of the site with which the high electrical resistance seal is to be provided, and wherein the step of positioning one or more ion channel-containing structures at one or more sites comprises the step of subjecting an internal volume of the passage of a selected site to a suction generating a flow of carrier liquid through the passage for guiding ion channel-containing structures towards the passage.

## Patentansprüche

1. Baueinheit, die folgendes umfasst:
ein ebenes Substrat (12), das einen ersten Oberflächenbereich und einen gegenüber liegenden zweiten Oberflächenbereich besitzt, wobei der erste Oberflächenbereich eine Vielzahl von Plätzen (14) aufweist, von denen jeder geeignet ist, eine Ionenkanäle enthaltende Struktur (2) aufzunehmen, die in einer Flüssigkeit enthalten ist, und jeder von denen einen in ihm verlaufenden Durchgang (30) durch das Substrat aufweist, wobei der Durchgang den ersten Oberflächenbereich und den zweiten Oberflächenbereich miteinander verbindet und wobei der Durchgang Wände besitzt und so dimensioniert ist, dass er eine Ionenkanäle enthaltende Struktur hält,
eine Vielzahl von Messelektroden (16), jede von denen einem entsprechenden Platz zugeordnet ist,
eine oder mehrere Referenzelektroden (8), wobei die Messelektroden und die entsprechende Referenzelektrode oder Referenzelektroden Elektroden sind, die in der Lage sind, dann, wenn sie miteinander in einem elektrolytischen Kontakt stehen und wenn zwischen ihnen eine Potentialdifferenz angelegt wird, durch die Abgabe von Ionen durch die eine Elektrode und die Aufnahme von Ionen durch die andere Elektrode zwischen sich einen Strom fließen zu lassen,
**dadurch gekennzeichnet, dass**
jeder der Plätze (14) in der Lage ist, eine dichte Verbindung mit hohem elektrischem Widerstand zu schaffen, die zwischen einem Berührungsbereich der äußeren Oberfläche einer Ionenkanäle enthaltenden Struktur, die an diesem Platz gehalten wird, und dem umgebenden ersten Oberflächenbereich des Substrates oder längs der Wände des Durchgangs (30) zu bilden, wobei die dichte Verbindung dann, wenn sie ausgebildet ist, eine Domäne, die auf der einen Seite der dichten Verbindung definiert ist und von der Ionenkanäle enthaltenden Struktur (2) gebildet wird und in elektrolytischem Kontakt mit der Messelektrode steht, von einer Domäne zu trennen, die auf der gegenüberliegenden Seite der dichten Verbindung von der Ionenkanäle enthaltenden Struktur gebildet wird und in elektrolytischem Kontakt mit der entsprechenden Referenzelektrode (8) steht, wodurch ein Strom, der zwischen der Referenzelektrode (8) und entsprechenden Messelektroden (16) und durch die Ionenkanäle enthaltenden Struktur fließt, ermittelt und/oder überwacht werden kann,
wobei die Elektroden (16, 8) mit der Baueinheit integriert sind und wobei die Baueinheit weiterhin eine Vielzahl von Strömungskanalstrukturen umfasst, die in dem Substrat erzeugt sind, um Flüssigkeit an die Vielzahl von Plätzen zu liefern.

2. Baueinheit nach Anspruch 1, die weiterhin eine Leitungsanordnung (32) für ein Ansaugen umfasst, wobei die Leitungsanordnung die elektrische Verdrahtung für die Messelektrode umfasst.

3. Baueinheit nach Anspruch 1 oder 2, bei der jeder Durchgang (30) mit Ansaugeinrichtungen verbunden ist, um es zu ermöglichen, ein Innenvolumen des Durchgangs eines ausgewählten Platzes einer Ansaugwirkung zu unterwerfen, die einen Strom von Trägerflüssigkeit durch den Durchgang erzeugt, um Ionenkanäle enthaltende Strukturen zum Durchgang hin zu führen.

4. Baueinheit nach einem der Ansprüche 1 bis 3, bei der die Elektroden (16, 8) durch eine Waver-Bearbeitungstechnologie ausgebildet worden sind.

5. . Baueinheit nach einem der vorhergehenden Ansprüche, bei der das Substrat (12) ein Silizium-Substrat ist und der Oberflächenbereich des Platzes, mit dem die Dichtung mit hohem elektrischen Widerstand erzeugt werden soll, ein Silizium-Oberflächenbereich ist, und bei der die Elektroden durch ein Verfahren ausgebildet worden sind, das ein Abscheide-Fotolithographie-ÄtzVerfahren umfasst.

6. Baueinheit nach einem der vorhergehenden Ansprüche, bei der die Vielzahl von Plätzen (14) in einer regelmäßigen Anordnung auf dem ersten Oberflächenbereich des Substrats angeordnet ist.

7. Baueinheit nach Anspruch 6, bei der die regelmäßige Anordnung von Plätzen (14) wenigstens neun Plätze umfasst.

8. Baueinheit nach einem der vorhergehenden Ansprüche, bei der die Messelektroden (16) und die Referenzelektroden (8) Silber/Silberhalogenid-Elektroden sind.

9. Baueinheit nach einem der Ansprüche 1 bis 7, bei der die Messelektroden (16) und Referenzelektroden (8) Silber/Silberchlorid-Elektroden sind.

10. Baueinheit nach einem der vorhergehenden Ansprüche, die eine erste Schicht aus einem hydrophoben Material (26) umfasst, das auf oder über der Oberfläche des Substrates angeordnet ist, wobei diese erste Schicht nur Teile der Oberfläche des Substrates bedeckt.

11. Baueinheit nach Anspruch 10, bei der einer oder mehrere Plätze (14) in Teilen der Oberfläche des Substrates angeordnet sind, die von dieser ersten Schicht nicht bedeckt sind.

12. Baueinheit nach einem der vorhergehenden Ansprüche, die eine oder mehrere Senken umfasst, die sich in das Substrat (12) hinein erstrecken und Senkenöffnungen aufweisen, die in dem ersten Oberflächenbereich ausgebildet sind, wobei jede Senke einen Bodenteil und einen Seitenteil aufweist, wobei wenigstens einige der Plätze (14) des ersten Oberflächenbereichs im Bodenteil der Senken angeordnet sind, um Flüssigkeit an die Vielzahl von Plätzen so abzugeben, dass eine Senke und ein Durchgang gemeinsam einen Trichter bilden.

13. Baueinheit nach Anspruch 12, bei der die Vielzahl von Strömungskanalstrukturen es ermöglicht, zu einem oder mehreren Trichtern Flüssigkeit hinzuzufügen.

14. Baueinheit nach Anspruch 13, bei der die Vielzahl von Strömungskanalstrukturen einen Einströmdurchgang und einen Ausströmdurchgang zu bzw. von dem einen oder mehreren Trichter umfasst.

15. Baueinheit nach einem der Ansprüche 12 bis 14, bei der die Senken durch ein Verfahren ausgebildet worden sind, das ein Photolithographie-Ätz-Verfahren umfasst.

16. Baueinheit nach Anspruch 15, bei der das Substrat (12) ein Siliziumsubstrat ist und bei der die Senken als stumpfkegelige Pyramiden ausgebildet sind, deren Böden von den Senken-Öffnungen gebildet werden und deren Seitenteile eine Neigung von 54,7° besitzen.

17. Baueinheit nach einem der vorhergehenden Ansprüche, bei der die Querdimension des Durchgangs (30) 1 bis 5 µm beträgt.

18. Baueinheit nach einem der Ansprüche 12 bis 17, bei der eine Referenzelektrode (8) auf dem Seitenteil einer jeden Senke positioniert ist.

19. Baueinheit nach einem der vorhergehenden Ansprüche, bei der die Messelektrode (16), die jedem Platz zugeordnet ist, an dem entsprechenden Platz angeordnet ist.

20. Baueinheit nach einem der Ansprüche 1 bis 18, bei der die jedem Platz (14) zugeordnete Messelektrode (16) auf dem gegenüberliegenden zweiten Oberflächenbereich des Substrates (12) angeordnet ist.

21. Baueinheit nach Anspruch 20, bei der die einem jeden Platz (14) zugeordnete Messelektrode (16) in der Nähe einer Öffnung des an dem betreffenden Platz ausgebildeten Durchgangs angeordnet ist.

22. Baueinheit nach einem der vorhergehenden Ansprüche, die weiterhin für jeden der Plätze (14) eine elektronische Schaltung umfasst, die mit der entsprechenden Messelektrode (16) und der Referenzelektrode (8) oder einer der Referenzelektroden für die Erzeugung eines verstärkten Signals verbunden ist, das eine eindeutige Funktion eines durch Ionenkanäle zwischen den Elektroden fließenden Stroms ist.

23. Verfahren zur Ausbildung einer Ganzzellen-Messkonfiguration zum Ermitteln und/oder Überwachen einer elektrophysiologischen Eigenschaft eines oder mehrerer Ionenkanäle von einer oder mehreren Ionenkanäle enthaltenden Strukturen (2), wobei dieses Verfahren folgende Schritte umfasst:
Bereitstellen einer Baueinheit, die folgendes aufweist: ein ebenes Substrat, das einen ersten Oberflächenbereich und einen gegenüberliegenden zweiten Oberflächenbereich aufweist, wobei dieses Substrat eine Vielzahl von Plätzen (14) in dem ersten Oberflächenbereich des Substrates besitzt, von denen jeder geeignet ist, eine Ionenkanäle enthaltende Struktur zu halten und von denen jeder einen in ihm ausgebildeten Durchgang (30) durch das Substrat hindurch aufweist, der den ersten Oberflächenbereich und den zweiten Oberflächenbereich miteinander verbindet, wobei der Durchgang Wände besitzt und so dimensioniert ist, dass er eine Ionenkanäle enthaltende Struktur hält und eine dichte Verbindung mit hohem Widerstand zwischen der Ionenkanäle enthaltenden Struktur und dem Substrat um oder längs der Wände des Durchgangs herum bildet, und eine Vielzahl von Messelektroden (16), von denen jede einem entsprechenden Platz zugeordnet ist, und eine oder mehrere Referenzelektroden (8),
Zuführen einer Trägerflüssigkeit zu einem oder mehreren Plätzen unter Verwendung einer Vielzahl von Strömungskanalstrukturen, die in dem Substrat ausgebildet sind, wobei die Trägerflüssigkeit eine oder mehrere Ionenkanäle enthaltende Strukturen enthält und die Trägerflüssigkeit mit dem ersten Oberflächenbereich des Substrates in Berührung steht,
Positionieren wenigstens einer der Ionenkanäle enthaltenden Strukturen an einer entsprechenden Anzahl von Plätzen,
Ausbilden einer einen hohen elektrischen Widerstand aufweisenden dichten Verbindung zwischen einem Berührungsbereich der äußeren Oberfläche einer Ionenkanäle enthaltenden Struktur, die an dem Platz gehalten wird, und einem ersten Oberflächenbereich des Substrates um die Wände des Durchgangs herum oder längs dieser Wände, wobei die dichte Verbindung dann, wenn sie ausgebildet ist, eine Domäne, die auf der einen Seite der dichten Verbindung durch die Ionenkanäle enthaltende Struktur definiert ist und in elektrolytischem Kontakt mit der Messelektrode steht, von einer Domäne trennt, die auf der gegenüberliegenden Seite der dichten Verbindung definiert ist und von der Ionenkanäle enthaltenden Struktur gebildet wird und in elektrolytischem Kontakt mit der entsprechenden Referenzelektrode steht,
Überprüfen einer dichten Verbindung mit hohem elektrischen Widerstand zwischen einer Ionenkanäle enthaltenden Struktur (2), die an einem Platz (14) in dem ersten Oberflächenbereich des Substrates oder längs der Wände des besagten Durchgangs gehalten wird, mit dem die den hohen elektrischen Widerstand besitzende dichte Verbindung ausgebildet werden soll, durch nacheinander erfolgendes Anlegen einer ersten elektrischen Potentialdifferenz zwischen der Messelektrode (16), die dem Platz zugeordnet ist, und einer Referenzelektrode (8), Überwachen eines ersten Stroms, der zwischen der Messelektrode und der Referenzelektrode fließt, und Vergleichen dieses ersten Stromes mit einem vorbestimmten Schwellenstrom und dann, wenn der erste Strom im wesentlichen der vorbestimmte Schwellenstrom ist, Zulassen des Platzes als Platz, der eine akzeptable dichte Verbindung zwischen der Ionenkanäle enthaltenden Struktur und dem ersten Oberflächenbereich des Platzes aufweist, und
Ausbilden einer Ganzzellen-Konfiguration an zugelassenen Plätzen,
wodurch ein dritter Strom, der durch Ionenkanäle der Ionenkanäle enthaltenden Struktur zwischen der Messelektrode und den Referenzelektroden fließt, bestimmt und/oder überwacht werden kann.

24. Verfahren nach Anspruch 23, bei dem der Schritt der Ausbildung einer Ganzzellen-Konfiguration an zugelassenen Plätzen das Anlegen einer Reihe von Impulsen mit einer zweiten elektrischen Potentialdifferenz zwischen der Messelektrode (16), die jedem zugelassenen Platz zugeordnet ist, und einer Referenzelektrode (8) umfasst, sowie das Überwachen eines zweiten Stroms, der zwischen der Messelektrode und der Referenzelektrode fließt, und Unterbrechen der Reihe von Impulsen mit einer zweiten elektrischen Potentialdifferenz immer dann, wenn der zweite Strom einen vorbestimmten Schwellenwert überschreitet, wodurch der Teil der Ionenkanäle enthaltenden Struktur zerrissen wird, der sich am nächsten an der Messelektrode befindet.

25. Verfahren nach Anspruch 23 oder 24, bei dem der Schritt der Ausbildung einer Ganzzellen-Konfiguration an zugelassenen Plätzen das Unterwerfen des Teils der Ionenkanäle enthaltenden Struktur, der sich am nächsten an der Messelektrode befindet, einer Wechselwirkung mit einer Porenbildenden Substanz umfasst.

26. Verfahren nach einem der Ansprüche 23 bis 25 und jedem hiervon abhängigem Anspruch, bei dem das Substrat an jedem der Plätze einen Durchgang aufweist, der den ersten und den zweiten Oberflächenbereich miteinander verbindet, wobei der Durchgang im wesentlichen in einem mittleren Teil des Oberflächenbereichs des Platzes angeordnet ist, mit dem die dichte Verbindung mit dem hohen elektrischen Widerstand ausgebildet werden soll, und bei dem der Schritt des Positionierens einer oder mehrerer Ionenkanäle enthaltenden Strukturen an einem oder mehreren Plätzen den Schritt umfasst, ein Innenvolumen des Durchgangs eines ausgewählten Platzes einer Saugwirkung auszusetzen, die einen Strom von Trägerflüssigkeit durch den Durchgang erzeugt, um Ionenkanäle enthaltenden Strukturen zum Durchgang hin zu führen.

## Revendications

1. Assemblage comprenant :
- un substrat plan (12) ayant une première partie de surface et une deuxième partie de surface opposée, la première partie de surface ayant une pluralité de sites (14) qui sont chacun adaptés pour retenir une structure contenant des canaux ioniques (2) contenue dans un liquide, et chacun ayant un passage (30) traversant le substrat, le passage reliant la première partie de surface et la deuxième partie de surface, lequel passage a des parois et est dimensionné pour retenir une structure contenant des canaux ioniques,
- une pluralité d'électrodes de mesure (16) qui sont chacune associées à un site respectif,
- une ou plusieurs électrodes de référence (8), les électrodes de mesure et l'électrode de référence ou les électrodes de référence respectives étant des électrodes capables, lorsqu'elles sont en contact électrolytique les unes avec les autres et lorsqu'une différence de potentiel est appliquée entre elles, de faire passer un courant entre elles sous l'effet d'un don d'ions par une électrode et d'une réception d'ions par l'autre électrode,
**caractérisé en ce que** :
- chacun des sites (14) est adapté pour fournir un scellement à résistance électrique élevée établi entre une zone de contact de la surface externe d'une structure contenant des canaux ioniques retenue sur le site et la première partie de surface sur le substrat, autour ou le long des parois dudit passage (30), le scellement, lorsqu'il est établi, séparant un domaine défini, sur un côté du scellement établi, par la structure contenant des canaux ioniques (2) et en contact électrolytique avec l'électrode de mesure, d'un domaine défini, sur le côté opposé du scellement établi, par la structure contenant des canaux ioniques et en contact électrolytique avec l'électrode de référence (8) respective, si bien qu'un courant passant entre les électrodes de référence (8) et de mesure (16) respective et traversant la structure contenant des canaux ioniques peut être déterminé et/ou contrôlé,
les électrodes (16,8) étant intégrées à l'assemblage, dans lequel l'assemblage comprend en outre une pluralité de structures à canaux d'écoulement formées dans le substrat pour distribuer un liquide dans la pluralité de sites.

2. Assemblage selon la revendication 1, définissant en outre une canalisation (32) pour la mise en oeuvre d'une aspiration, la canalisation comprenant un câblage électrique pour l'électrode de mesure.

3. Assemblage selon la revendication 1 ou selon la revendication 2, dans lequel chaque passage (30) est relié à un moyen d'aspiration pour pouvoir soumettre un volume interne du passage d'un site choisi à une aspiration produisant un écoulement de liquide véhicule à travers le passage pour guider les structures contenant des canaux ioniques vers le passage.

4. Assemblage selon l'une quelconque des revendications 1 à 3, dans lequel les électrodes (16, 8) ont été formées au moyen d'une technique de traitement des plaquettes.

5. Assemblage selon l'une quelconque des revendications précédentes, dans lequel le substrat (12) est un substrat de silicium, et la partie de surface du site avec laquelle le scellement à résistance électrique élevée doit être établi est une partie de surface de silice, et les électrodes ont été formées par un procédé comprenant des étapes de dépôt/photolithographie/gravure.

6. Assemblage selon l'une quelconque des revendications précédentes, dans lequel la pluralité de sites (14) est disposée en matrice sur la première partie de surface du substrat.

7. Assemblage selon la revendication 6, dans lequel la matrice de sites (14) comprend au moins 9 sites.

8. Assemblage selon l'une quelconque des revendications précédentes, dans lequel les électrodes de mesure (16) et de référence (8) sont des électrodes argent/halogénure d'argent.

9. Assemblage selon l'une quelconque des revendications 1 à 7, dans lequel les électrodes de mesure (16) et de référence (8) sont des électrodes argent/chlorure d'argent.

10. Assemblage selon l'une quelconque des revendications précédentes, comprenant une première couche de matière hydrophobe (26) positionnée sur ou au-dessus de la surface du substrat, ladite première couche ne recouvrant que des parties de la surface du substrat.

11. Assemblage selon la revendication 10, dans lequel un ou plusieurs sites (14) son situés dans des parties de la surface du substrat non recouvertes par ladite première couche.

12. Assemblage selon l'une quelconque des revendications précédentes, comprenant un ou plusieurs puits s'étendant dans le substrat (12) et ayant des ouvertures de puits définies dans la première partie de surface, ayant chacun une partie inférieure et une partie latérale, au moins une partie des sites (14) de la première partie de surface étant positionnés dans les parties inférieures des puits pour distribuer un liquide à ladite pluralité de sites, de sorte qu'un puits et un passage définissent ensemble un entonnoir.

13. Assemblage selon la revendication 12, dans lequel la pluralité de structures à canaux d'écoulement permet d'ajouter un liquide à un ou plusieurs entonnoirs.

14. Assemblage selon la revendication 13, dans lequel la pluralité de structures à canaux d'écoulement comprend une arrivée et une sortie d'écoulement allant vers/venant d'un ou plusieurs entonnoirs.

15. Assemblage selon l'une quelconque des revendications 12 à 14, dans lequel les puits ont été formés par un procédé comprenant des étapes de photolithographie/gravure.

16. Assemblage selon la revendication 15, dans lequel le substrat (12) est un substrat de silicium, et dans lequel les puits sont sous forme de pyramides tronquées dont les surfaces inférieures sont constituées par les ouvertures de puits et dont les parties latérales ont une inclinaison de 54,7°.

17. Assemblage selon l'une quelconque des revendications précédentes, dans lequel la dimension transversale du passage (30) est de 1 à 5 µm.

18. Assemblage selon l'une quelconque des revendications 12 à 17, dans lequel une électrode de référence (8) est positionnée sur la partie latérale de chaque puits.

19. Assemblage selon l'une quelconque des revendications précédentes, dans lequel l'électrode de mesure (16) associée à chaque site est positionnée sur chaque site respectif.

20. Assemblage selon l'une quelconque des revendications 1 à 18, dans lequel l'électrode de mesure (16) associée à chaque site (14) est positionnée sur la deuxième partie de surface opposée du substrat (12).

21. Assemblage selon la revendication 20, dans lequel l'électrode de mesure (16) associée à chaque site (14) est positionnée à côté d'une ouverture du passage défini sur le site respectif.

22. Assemblage selon l'une quelconque des revendications précédentes, comprenant en outre, pour chacun des sites (14), un circuit électronique qui est relié à l'électrode de mesure (16) respective et à l'électrode de référence (8) ou à l'une des électrodes de référence pour la production d'un signal amplifié qui est une fonction unique d'un courant traversant les canaux ioniques entre lesdites électrodes.

23. Procédé d'établissement d'une configuration de mesure de cellules entières pour déterminer et/ou contrôler une propriété électrophysiologique d'un ou plusieurs canaux ioniques d'une ou plusieurs structures contenant des canaux ioniques (2), ledit procédé comprenant les étapes de :
- fourniture d'un assemblage comprenant : un substrat plan ayant une première partie de surface et une deuxième partie de surface opposée, ledit substrat ayant une pluralité de sites (14) dans la première partie de surface du substrat, qui sont chacun adaptés pour retenir une structure contenant des canaux ioniques et qui ont chacun un passage (30) traversant le substrat, reliant la première partie de surface et la deuxième partie de surface, lequel passage a des parois et est dimensionné pour retenir une structure contenant des canaux ioniques et pour former un scellement à haute résistance entre ladite structure contenant des canaux ioniques et ledit substrat, autour ou le long des parois dudit passage, une pluralité d'électrodes de mesure (16) qui sont chacune associées à un site respectif, et une ou plusieurs électrodes de référence (8) ;
- distribution d'un liquide véhicule sur un ou plusieurs sites à l'aide d'une pluralité de structures à canaux d'écoulement formées dans le substrat, ledit liquide véhicule contenant une ou plusieurs structures contenant des canaux ioniques, le liquide véhicule étant en contact avec la première partie de surface du substrat ;
- positionnement d'au moins une des structures contenant des canaux ioniques sur un nombre correspondant de sites ;
- formation d'un scellement à résistance électrique élevée entre une zone de contact de la surface externe d'une structure contenant des canaux ioniques retenue sur le site et une première partie de surface du substrat, autour ou le long des parois dudit passage, le scellement, lorsqu'il est établi, séparant un domaine défini, sur un côté du scellement établi, par la structure contenant des canaux ioniques et en contact électrolytique avec l'électrode de mesure, d'un domaine défini, sur le côté opposé du scellement établi, par la structure contenant des canaux ioniques et en contact électrolytique avec l'électrode de référence respective ;
- vérification d'un scellement à résistance électrique élevée entre une structure contenant des canaux ioniques (2) retenue sur un site (14) dans la première partie de surface du substrat ou le long des parois dudit passage avec laquelle le scellement à résistance électrique élevée doit être établi, en appliquant successivement une première différence de potentiel électrique entre l'électrode de mesure (16) associée au site et une électrode de référence (8), en contrôlant un premier courant passant entre ladite électrode de mesure et ladite électrode de référence, et en comparant ledit premier courant à un courant seuil prédéterminé et, si le premier courant est tout au plus le courant seuil prédéterminé, en approuvant alors le site comme ayant un scellement acceptable entre la structure contenant des canaux ioniques et la première partie de surface du site ; et
- établissement d'une configuration à cellules entières sur les sites approuvés,
si bien qu'un troisième courant traversant les canaux ioniques de la structure contenant des canaux ioniques entre l'électrode de mesure et les électrodes de référence peut être déterminé et/ou contrôlé.

24. Procédé selon la revendication 23, dans lequel l'étape d'établissement d'une configuration à cellules entières sur les sites approuvés comprend l'application, entre l'électrode de mesure (16) associée à chaque site approuvé et une électrode de référence (8), d'une série d'impulsions d'une deuxième différence de potentiel électrique, le contrôle d'un deuxième courant passant entre l'électrode de mesure et l'électrode de référence, et l'interruption de la série d'impulsions d'une deuxième différence de potentiel électrique lorsque ledit deuxième courant dépasse une valeur seuil prédéterminée, rompant ainsi la partie de la structure contenant des canaux ioniques qui est la plus proche de l'électrode de mesure.

25. Procédé selon la revendication 23 ou selon la revendication 24, dans lequel l'étape d'établissement d'une configuration à cellules entières sur les sites approuvés comprend la soumission de la partie de la structure contenant des canaux ioniques qui est la plus proche de l'électrode de mesure à une interaction avec une substance porogène.

26. Procédé selon l'une quelconque des revendications 23 à 25 et l'une quelconque des revendications qui en dépendent, dans lequel le substrat définit, sur chacun des sites, un passage reliant les première et deuxième parties de surface, le passage étant positionné essentiellement dans une partie centrale de la partie de surface du site avec laquelle le scellement à résistance électrique élevée doit être fourni, et dans lequel l'étape de positionnement d'une ou plusieurs structures contenant des canaux ioniques sur un ou plusieurs sites comprend l'étape consistant à soumettre un volume interne du passage d'un site choisi à une aspiration produisant un écoulement de liquide véhicule à travers le passage pour guider la structure contenant des canaux ioniques vers le passage.
